# EUROPÄISCHE PATENTSCHRIFT

(11) **EP 4 529 853 B1**
(45) Veröffentlichungstag und Bekanntmachung des Hinweises auf die Patenterteilung: **18.02.2026**
(21) Anmeldenummer: 23199724.8
(22) Anmeldetag: 26.09.2023
(51) Int. Cl.: A61B 6/42, G01T 1/00

(54) **BILDGEBUNGSSYSTEM UND VERFAHREN ZUR AUFNAHME VON PROJEKTIONSBILDERN**
IMAGING SYSTEM AND METHOD FOR CAPTURING PROJECTION IMAGES
SYSTÈME D'IMAGERIE ET PROCÉDÉ D'ACQUISITION D'IMAGE DE PROJECTION

(43) Veröffentlichungstag der Anmeldung: 02.04.2025
(73) Patentinhaber: Siemens Healthineers AG, 91301 Forchheim (DE)
(72) Erfinder: Ergler, Thorsten, 91054 Erlangen (DE); Schaffert, Stefan, 91058 Erlangen (DE)
(74) Vertreter: Siemens Healthineers Patent Attorneys

(56) Entgegenhaltungen:
- US-A1- 2009 129 538
- SU TING ET AL: "Super resolution dual-energy cone-beam CT imaging with dual-layer flat-panel detector", ARXIV (CORNELL UNIVERSITY), 18 October 2022 (2022-10-18), Ithaca, XP093120527, Retrieved from the Internet <URL:https://arxiv.org/pdf/2210.05884.pdf> [retrieved on 20240117], DOI: 10.48550/arxiv.2210.05884
- SHI LINXI ET AL: "Single-shot quantitative x-ray imaging using a primary modulator and dual-layer detector: simulation and phantom studies", PROGRESS IN BIOMEDICAL OPTICS AND IMAGING, SPIE - INTERNATIONAL SOCIETY FOR OPTICAL ENGINEERING, BELLINGHAM, WA, US, vol. 12031, 4 April 2022 (2022-04-04), pages 1203106 - 1203106, XP060155815, ISSN: 1605-7422, ISBN: 978-1-5106-0027-0, DOI: 10.1117/12.2611591

## Beschreibung

Die Erfindung betrifft ein Bildgebungssystem und ein Verfahren zur Aufnahme von Projektionsbildern.

In einem Röntgenbild, womit ein mittels Röntgenstrahlen aufgenommenes Projektionsbild gemeint ist, gibt es außerhalb des Objektbereichs einen Bereich, der mit Primärstrahlung und Streustrahlung vom Objekt bestrahlt wird. Da dieser Bereich mit zunehmendem seitlichen Abstand zum Objekt abnimmt, sind die höchsten Strahlenintensitäten typischerweise am Übergang Haut-Luft zu finden.

Röntgendetektoren weisen eine Sättigung auf, wenn die Intensität der einfallenden Röntgenstrahlung (die Primärstrahlung) den Dynamikbereich des Detektors überschreitet. Gesättigte Pixelbereiche können nicht mehr für die Bildanalyse oder Diagnose verwendet werden. Normalerweise werden gesättigte Pixel sogar abgeschnitten. Der Dynamikbereich wird durch die minimal erforderliche Empfindlichkeit (digitale Auflösung des einfallenden Röntgensignals) und das maximal akzeptable Röntgensignal aufgrund elektronischer Einschränkungen (Bittiefe, maximale Speicherkapazität) begrenzt.

Bei der Mammographie verschärft sich die Situation im Vergleich zur Radiographie durch die hier verwendete erhöhte Objektdämpfung von Röntgenphotonenenergien unter 50 kVp. Die Intensität im Haut-Luft-Übergangsbereich kann sich um mehrere Größenordnungen ändern. Der Dynamikbereich des Detektors reicht nicht aus, um alle Intensitäten für alle beobachteten Objektdicken und -dichten in einem Bild zu erfassen.

Es wäre vorteilhaft, wenn dieser "Haut-zu-Luft-Übergang" vermessen werden könnte, da daraus nützliche Informationen gewonnen werden können, **z.B.** im Hinblick auf den Grad der Streustrahlung bzw. die einfallende Röntgenintensität. Die größte Herausforderung besteht darin, dass der Haut-zu-Luft-Übergang typischerweise nicht abgebildet wird, also nicht deutlich sichtbar gemacht werden kann, da der Sättigungsbereich des Detektors in den eigentlichen Bildbereich hineinragt.

Ein weiterer wichtiger Aspekt ist die Bestimmung der gesamten applizierten Dosis mit hoher Präzision. Dies ist derzeit aufgrund der Detektorsättigung nicht möglich. Dies führt **z.B. zu** großen Fehlern bei der abgeleiteten Jodakkumulation in bestimmten Objektbereichen bei kontrastverstärkten Dual-Energy-Untersuchungen.

US 2009/129538 A1 beschreibt ein diagnostisches Bildgebungssystem mit einer Röntgenquelle, die einen Röntgenstrahl auf ein abzubildendes Objekt aussendet, und einen Energiediskriminator-Detektor (ED), der den Röntgenstrahl empfängt. Der ED umfasst eine erste Schicht mit einem Halbleiter, der für den Betrieb in einem integrierenden Modus konfigurierbar ist, und eine zweite Schicht mit einer größeren Dicke, die für den Empfang von Röntgenstrahlen konfiguriert ist, die durch die erste Schicht hindurchgehen.

Su Ting et al. beschreiben in ihrer Arbeit "Super resolution dual-energy cone-beam CT imaging with dual-layer flat-panel detector" (arXiv (Cornell University), 18. Oktober 2022 (2022-10-18), https://arxiv.org/pdf/2210.05884.pd) ein superauflösendes Dual-Energy-CBCT-Bildgebungsverfahren namens suRi, das auf einem Dual-Layer-FPD (DL-FPD) basiert und eine Erhöhung der Signalauslesegeschwindigkeit bei einer guten räumlichen Auflösung erreicht.

Eine höhere Pixelspeicherkapazität im Detektor kann den Bereich des einfallenden Röntgensignals für die Digitalisierung erweitern. Dies geht jedoch mit einem nachteilhaften erhöhten Pixelrauschen einher.

Es könnten auch mehrere Bilder für dasselbe Objekt verwendet werden, um die gesamte klinisch verwendete Dosis in kleinere Teile aufzuteilen, die noch vom Detektor abgedeckt werden können. Dies wird jedoch auch durch ein erhöhtes Bildrauschen bei mehreren Auslesungen und das mögliche Auftreten von Bewegungsartefakten aufgrund der längeren Gesamterfassungsdauer negativ beeinflusst.

Es ist eine Aufgabe der vorliegenden Erfindung, ein Bildgebungssystem und ein Verfahren zur Aufnahme von Projektionsbildern anzugeben, mit dem die oben beschriebenen Nachteile vermieden werden.

Diese Aufgabe wird durch ein Bildgebungssystem gemäß Patentanspruch 1 und ein Verfahren gemäß Patentanspruch 7 gelöst.

Ein erfindungsgemäßes Bildgebungssystem dient zur Aufnahme von Projektionsbildern. Das Bildgebungssystem umfasst eine Detektoreinheit und eine Strahlungsquelle, wobei die Detektoreinheit zwei hintereinander angeordnete Detektorelemente aufweist, die beide denselben Aufnahmebereich überdecken. Zudem ist die Strahlungsquelle so eingestellt und/oder die Detektoreinheit so ausgestaltet, dass für Teile des Aufnahmebereichs, in denen bei einer Bestrahlung das erste Detektorelement in Sättigung ist, das zweite Detektorelement nicht in Sättigung ist. Zusätzlich umfasst das Bildgebungssystem eine Datenakquisitionseinheit, die dazu ausgelegt ist, einen ersten Datensatz des ersten Detektorelements aufzunehmen und einen zweiten Datensatz des zweiten Detektorelements aufzunehmen, so dass beide Datensätze voneinander trennbar sind.

Im Grunde kann als Bildgebungssystem ein normales Radiographiesystem, Fluoroskopiesystem oder Urologiesystem aber auch ein System zur Tomographie oder Tomosynthese verwendet werden. Es müsste jedoch mit einer Detektoreinheit ausgestattet sein, welche zwei hintereinander angeordnete Detektorelemente aufweist. Im Grunde ist die Erfindung für alle Anwendungen von Vorteil, bei denen Projektionsbilder aufgenommen werden, egal ob diese das Resultat sind oder zur Rekonstruktion von Bildern verwendet werden.

Die Detektorelemente müssen dabei so angeordnet sein, dass ein Röntgenstrahl gleichzeitig auf beide Detektorelemente fällt, diese also beide denselben Aufnahmebereich überdecken. Hier könnte die Auswirkung eines kegelförmigen Strahls auf Unterschiede in den Aufnahmen der beiden Detektorelemente korrigiert werden. Eine solche Korrektur ist im Stand der Technik bekannt.

Damit das Verfahren vorteilhaft angewandt werden kann, werden die Daten des zweiten Detektorelements benötigt. Dieses darf also zumindest in den Bereichen, deren Daten verwendet werden, nicht in Sättigung sein. Ob das zweite Detektorelement in Sättigung geht, hängt zum einen von der Intensität von der Primärstrahlung ab und zum anderen von der abschirmenden Wirkung des ersten Detektorelements und ggf. weiterer Absorber. Aus diesem Grund muss die Strahlungsquelle so eingestellt sein bzw. die Detektoreinheit so ausgestaltet sein, dass für Teile des Aufnahmebereichs, in denen bei einer Bestrahlung das erste Detektorelement in Sättigung ist, das zweite Detektorelement nicht in Sättigung ist. Dies müssen wie gesagt nicht alle Bereiche des zweiten Detektorelements sein, aber es sollten die Bereiche eines Übergangs eines Objekts zur Luft sein, da diese am interessantesten sind.

Durch die Verwendung des zweiten Detektorelements hinter dem ersten Detektorelement ist es also möglich, die Röntgenstrahlen zu detektieren, welche das erste Detektorelement durchdrungen haben. Die auf das zweite Detektorelement auftreffende Strahlung hat durch Absorption im ersten Detektorelement eine geringere Intensität, so dass das zweite Detektorelement weniger, überwiegend hochenergetische Photonen empfängt. Bei richtiger Einstellung der Röntgenquelle kann das zweite Detektorelement das Direkt- und Streustrahlungsmuster vollständig erfassen und eine Sättigung des ersten Detektorelements kompensieren, ohne dass Einbußen in der Qualität des Originalbilds in Kauf genommen werden müssen.

Geeignete Datenakquisitionseinheiten für das Bildgebungssystem sind im Grunde bekannt. Für die Erfindung muss die Datenakquisitionseinheit jedoch so gestaltet sein, dass der erste Datensatz des ersten Detektorelements von dem zweiten Datensatz des zweiten Detektorelements trennbar ist. Die Datensätze sollten dabei getrennt voneinander abgespeichert werden oder zumindest Markierungen aufweisen, die eine eindeutige Zuordnung ermöglichen.

Das Bildgebungssystem umfasst zusätzlich eine Bilderstellungseinheit, ausgelegt zur Erstellung eines Projektionsbildes, dessen erster Bereich, insbesondere ein zentraler Bereich, durch Daten des ersten Datensatzes erstellt wird, und dessen zweiter Bereich, insbesondere ein Randbereich, durch Daten des zweiten Datensatzes oder Daten beider Datensätze erstellt wird, wobei Bilddaten, bei denen das erste Detektorelement in Sättigung war, mittels des zweiten Datensatzes rekonstruiert werden.

Das Bildgebungssystem ist dabei dazu ausgelegt, Position und Signalverlauf an einer Oberfläche eines aufgenommenen Objekts aus dem zweiten Datensatz zu extrahieren und diese Informationen für eine Bestimmung eines entsprechenden Signalverlaufs aus dem ersten Datensatz zu verwenden.

Ein erfindungsgemäßes Verfahren dient zur Aufnahme von Projektionsbildern mit einem Bildgebungssystem nach einem der vorangehenden Ansprüche. Es umfasst die folgenden Schritte:
- Bestrahlen eines Körperteils eines Patienten mit der Strahlungsquelle, wobei beide Detektorelemente der Detektoreinheit durch das Körperteil hindurch bestrahlt werden,
- Auslesen beider Detektorelemente und Bilden eines ersten Datensatzes durch Auslese des ersten Detektorelements und eines zweiten Datensatzes durch Auslese des zweiten Detektorelements,
- Erstellung eines Projektionsbildes aus beiden Datensätzen, wobei Bildwerte im Projektionsbild, in denen das erste Detektorelement beim Auslesen in Sättigung war, auf Daten aus dem zweiten Datensatz basieren.

Es werden dabei Position und Signalverlauf an einer Oberfläche eines aufgenommenen Objekts aus dem zweiten Datensatz extrahiert und diese Informationen für eine Bestimmung eines entsprechenden Signalverlaufs aus dem ersten Datensatz verwendet.

Die Aufnahme von Projektionsbildern durch Bestrahlung ist hinlänglich bekannt. Eine Besonderheit des Verfahrens ist, dass hier beide Detektorelemente ausgelesen werden und mit dem zweiten Datensatz Informationen ermittelt, **z.B.** ergänzt, werden, in Bereichen, in denen das erste Detektorelement in Sättigung war.

Weitere, besonders vorteilhafte Ausgestaltungen und Weiterbildungen der Erfindung ergeben sich aus den abhängigen Ansprüchen sowie der nachfolgenden Beschreibung, wobei die Ansprüche einer Anspruchskategorie auch analog zu den Ansprüchen und Beschreibungsteilen zu einer anderen Anspruchskategorie weitergebildet sein können und insbesondere auch einzelne Merkmale verschiedener Ausführungsbeispiele bzw. Varianten zu neuen Ausführungsbeispielen bzw. Varianten kombiniert werden können.

Bei einer bevorzugten Ausführungsform ist die Strahlungsquelle eine Röntgenstrahlenquelle oder Teilchenstrahlenquelle. Das Bildgebungssystem ist bevorzugt ein medizintechnisches Bildgebungssystem, **z.B.** ein Radiographiesystem, insbesondere für die Mammographie.

Ein bevorzugtes Bildgebungssystem umfasst eine Korrektureinheit ausgelegt zur Korrektur zumindest eines der Datensätze. Die Korrektureinheit ist dabei bevorzugt zu mindestens einer der folgenden Korrekturen ausgelegt: Rauschunterdrückung, Pixelregistrierung, Korrektur der Kegelstrahlvergrößerung und/oder spektrale Korrektur. Solche Korrektureinheiten, insbesondere in Form von Softwaremodulen sind an sich bekannt. Ihr Einsatz für das erfindungsgemäße Verfahren ist vorteilhaft. Es kann Rechenzeit gespart werden, wenn die Korrekturen dort wo das erste Detektormodul in Sättigung war, nicht auf den ersten Datensatz angewandt werden. Es kann auch von Vorteil sein, dass Korrekturen dort wo der erste Datensatz zur Erstellung des Projektionsbildes verwendet wird die Korrekturen nicht auf den zweiten Datensatz anzuwenden, zumindest dann nicht, wenn der zweite Datensatz dazu nicht verwendet wird.

Ein erfindungsgemäßes Bildgebungssystem umfasst eine Bilderstellungseinheit, welche zur Erstellung eines Projektionsbildes ausgelegt ist. Sie ist genauer dazu ausgelegt, dass ein erster Bereich des Projektionsbildes, insbesondere ein zentraler Bereich, durch Daten des ersten Datensatzes erstellt wird. Es können dazu aber auch Daten des zweiten Datensatzes verwendet werden, **z.B.** wenn diese andere spektrale Informationen enthalten. Ein zweiter Bereich des Projektionsbildes, insbesondere ein Randbereich, wird durch Daten des zweiten Datensatzes oder durch Daten beider Datensätze erstellt. Dabei werden bevorzugt Bilddaten, bei denen das erste Detektorelement in Sättigung war, mittels Daten des zweiten Datensatzes, also des zweiten Detektorelements, rekonstruiert. Es können hierzu einfach die Daten des zweiten Datensatzes verwendet werden. Diese sind jedoch anders skaliert als die Daten des ersten Datensatzes, da das zweite Detektorelement eine andere Intensität erfuhr. Es ist jedoch möglich, die Daten des zweiten Datensatzes so zu skalieren, dass sie zu den Daten des ersten Datensatzes passen. Dazu werden Informationen des ersten Datensatzes verwendet, **z.B.** der Verlauf im Bereich des Objekts, für diese Daten eine Skalierungsfunktion ermittelt und diese Skalierungsfunktion auf alle Daten des zweiten Datensatzes angewendet (also auch dort, wo das erste Detektorelement in Sättigung war).

Bevorzugt ist das zweite Detektorelement dazu ausgelegt, höherenergetische Strahlung zu empfangen als das erste Detektorelement. Dies ist von Vorteil, da im ersten Detektorelement vorwiegend Strahlen mit niedriger Energie komplett absorbiert werden. Darüber hinaus erhält man dadurch spektrale Informationen, die zur Erstellung des Projektionsbildes verwendet werden können.

Bevorzugt ist das erste Detektorelement als Absorber ausgestaltet. Hierzu sollte beachtet werden, dass alle Materialien in einem Strahlengang absorbierend wirken. Gemeint ist hier, dass das Detektorelement über seine Funktion als Detektor hinaus als Absorber ausgestaltet ist, **z.B.** indem ein Substrat für das Detektormaterial dicker als normal ausgestaltet ist. Insbesondere ist die Detektionsschicht des Detektorelements oder eine Fläche in oder an einer Detektionsschicht so ausgestaltet ist, dass bei einer vorbestimmten Strahlintensität für eine Bildaufnahme das zweite Detektorelement nicht in Sättigung geht.

Bevorzugt umfasst die Detektoreinheit eine Absorberplatte zwischen dem ersten Detektorelement und dem zweiten Detektorelement, welche das zweite Detektorelement, zumindest teilweise, überdeckt, wobei die Absorberplatte bevorzugt entnehmbar oder austauschbar ist. Dadurch kann das zweite Detektorelement gezielt abgeschirmt werden, dass es nicht in Sättigung geht.

Gemäß einer bevorzugten Ausführungsform des Verfahrens wird ein erster Bereich des Projektionsbildes aus dem ersten Datensatz (den Informationen des oberen Detektorelements) erstellt. Dieser erste Bereich zeigt das Objekt, **z.B.** das Körperteil, und ist insbesondere ein zentraler Bereich des Projektionsbildes. Er kann auch unter Zuhilfenahme des zweiten Datensatzes erstellt werden, insbesondere wenn dieser weitere spektrale Informationen umfasst.

Ein zweiter Bereich ist insbesondere ein Randbereich um einen zentralen Bereich herum und wird aus dem zweiten Datensatz oder einer Kombination beider Datensätze erstellt. Bei letzterem wird der erste Datensatz bevorzugt zur Anpassung des zweiten Datensatzes an den ersten Datensatz verwendet.

Bevorzugt wird der zweite Bereich, insbesondere ein Randbereich, durch eine gewichtete Faltung beider Datensätze oder durch Skalierung gebildet. Dies kann insbesondere durch Verwendung eines Faltungskerns oder durch Spektralfilterung mit einer anschließenden Wichtung von Daten des zweiten Datensatzes mit den entsprechenden Daten des ersten Datensatzes erfolgen.

Besonders bevorzugt wird aus dem zweiten Datensatz und dem ersten Datensatz eine Gesamtdosis über das gesamte Bild ermittelt. Dies kann **z.B.** dadurch erfolgen, dass zunächst die vom zweiten Detektorelement gemessene Intensität im zweiten Bereich, also dort wo das erste Detektorelement in Sättigung war, mittels der Daten des ersten Datensatzes auf den ersten Datensatz angepasst. Dann würden die Daten im zweiten Bereich den Fall wiedergeben, in dem das erste Detektorelement dort nicht in Sättigung gewesen wäre. Aus dem Verlauf dieser Daten im zweiten Bereich kann nun auf die Gesamtintensität geschlossen und über die gesamte Detektorfläche extrapoliert werden. Dies kann mittels durch Testmessungen ohne Motiv festgelegter Formeln oder unter Zuhilfenahme der System-Korrekturtabellen erfolgen.

Gemäß der erfindungsgemäßen Ausführungsform des Verfahrens werden Position und Signalverlauf an einer Oberfläche eines aufgenommenen Objekts aus dem zweiten Datensatz extrahiert. Dies sind insbesondere Position und Signalverlauf eines Haut-Luft-Übergangs, **z.B.** an einer Brust. Diese Informationen werden dann für eine Bestimmung eines entsprechenden Signalverlaufs aus dem ersten Datensatz verwendet. Bevorzugt wird dazu eine seitliche Grenze eines aufgenommenen Objekts, insbesondere ein Haut-Luft Übergang, basierend auf dem zweiten Datensatz bestimmt und/oder korrigiert. Hier sollte beachtet werden, dass eine Sättigung des ersten Detektorelements dort Informationen über den Haut-Luft Übergang "verwischt".

Bevorzugt wird zumindest einer der Datensätze im Hinblick auf geometrische Effekte im Zusammenhang mit einer Dual-Layer-Technologie korrigiert. Dabei wird bevorzugt eine Pixelregistrierung des ersten Datensatzes auf den zweiten Datensatz durchgeführt oder umgekehrt. Alternativ oder zusätzlich kann auch eine Korrektur der Kegelstrahlvergrößerung durchgeführt werden. Hier sollte beachtet werden, dass sich die beiden Detektorelemente in unterschiedlichen Abständen vom Objekt und der Quelle befinden und typischerweise ein kegelförmiger Strahl verwendet wird.

Bevorzugt werden die aufgenommenen Datensätze spektral korrigiert, insbesondere basierend auf zuvor erfassten Kalibrierungsdaten. Bevorzugt wird dabei eine Korrektur der Strahlaufhärtung durchgeführt. Damit können Strahlaufhärtungseffekte aufgrund des Doppelschichtdesigns der Detektoreinheit kompensiert werden. Es kann dazu insbesondere A-Priori-Wissen verwendet werden. Da die Strahlaufhärtung der Detektoreinheit bekannt ist, kann dies zur Korrektur verwendet werden.

Die Erfindung wird im Folgenden unter Hinweis auf die beigefügten Figuren anhand von Ausführungsbeispielen noch einmal näher erläutert. Dabei sind in den verschiedenen Figuren gleiche Komponenten mit identischen Bezugsziffern versehen. Die Figuren sind in der Regel nicht maßstäblich. Es zeigen:
Figur 1 ein Bildgebungssystem zur Aufnahme von Projektionsbildern,
Figur 2 Daten einer Aufnahme gemäß dem Stand der Technik,
Figur 3 Datensätze einer Aufnahme gemäß der Erfindung,
Figur 4 ein Verfahren zur Aufnahme von Projektionsbildern mit einem erfindungsgemäßen Bildgebungssystem.

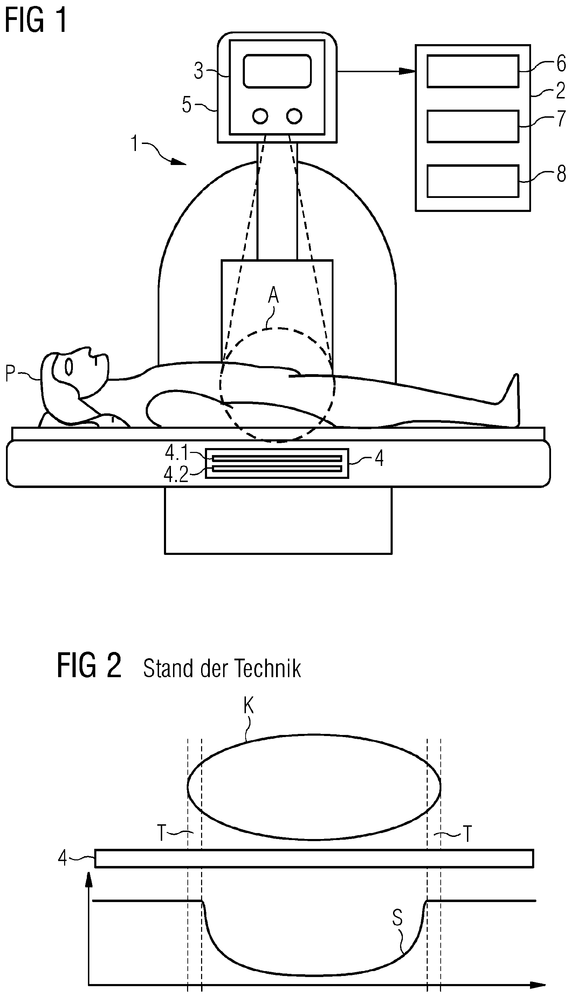

Figur 1 zeigt grob schematisch ein Bildgebungssystem 1 in Form eines Radiographie-Systems 1 mit einer Steuereinrichtung 2 zur Aufnahme von Projektionsbildern. Das RadiographieSystem 1 weist in üblicher Weise eine Strahlungsquelle 3 auf, welche hier eine Röntgenquelle darstellt, und während einer Radiographieaufnahme einen Patienten P durchstrahlt, so dass die Strahlung auf eine der Strahlungsquelle gegenüberliegende Detektoreinheit 4 trifft.

Die Detektoreinheit 4 umfasst zwei hintereinander angeordnete Detektorelemente 4.1, 4.2, die beide denselben Aufnahmebereich A überdecken. Die Strahlungsquelle 3 ist dabei so eingestellt und die Detektoreinheit 4 so ausgestaltet, dass für Teile des Aufnahmebereichs A, in denen bei einer Bestrahlung das erste Detektorelement 4.1 in Sättigung ist, das zweite Detektorelement 4.2 nicht in Sättigung ist. Dies kann z.B. mit einer Vorabaufnahme des Patienten P geschehen.

Bei der Steuereinrichtung 2 sind nur die Komponenten dargestellt, die für die Erläuterung der Erfindung wesentlich sind. Grundsätzlich sind Radiographie-Systeme 1 und zugehörige Steuereinrichtungen 2 dem Fachmann bekannt und brauchen daher nicht im Detail erläutert zu werden.

Die Steuereinrichtung 2 umfasst eine Datenakquisitionseinheit 6, die dazu ausgelegt ist, einen ersten Datensatz D1 des ersten Detektorelements 4.1 aufzunehmen und einen zweiten Datensatz D2 des zweiten Detektorelements 4.2 aufzunehmen, so dass beide Datensätze D1, D2 voneinander trennbar sind.

Zusätzlich umfasst die Steuereinrichtung 2 eine Korrektureinheit 7 ausgelegt zur Korrektur zumindest eines der Datensätze D1, D2. Die Korrektureinheit 7 ist z.B. zur Rauschunterdrückung, zur Pixelregistrierung, zu einer Korrektur der Kegelstrahlvergrößerung und/oder zu einer spektralen Korrektur ausgelegt.

Des Weiteren umfasst die Steuereinrichtung 2 eine Bilderstellungseinheit 8 ausgelegt zur Erstellung eines Projektionsbildes, dessen erster Bereich, insbesondere ein zentraler Bereich, durch Daten des ersten Datensatzes D1 erstellt wird, und insbesondere auch durch Daten des zweiten Datensatzes D2, und dessen zweiter Bereich, insbesondere ein Randbereich, durch Daten des zweiten Datensatzes D2 oder Daten beider Datensätze D1, D2 erstellt wird.

Figur 2 zeigt Daten einer Aufnahme gemäß dem Stand der Technik. Ein Körperbereich K, z.B. eine Brust, wird von oben mit Röntgenstrahlung bestrahlt. Die Strahlung trifft auf die Detektoreinheit 4, die hier aus einem einzelnen Element gebildet wird und es wird von der Detektoreinheit ein Signal S detektiert, dessen Verlauf in Form eines Graphen wiedergegeben ist. Dort wo der Körperteil K ist, ist das detektierte Signal S niedrig und steigt zum Rand hin an. Im Bereich des Haut-Luft-Übergangs T steigt dann das Signal S so stark an, dass die Detektoreinheit in Sättigung geht, was mit einem horizontalen Verlauf des Signals angedeutet ist.

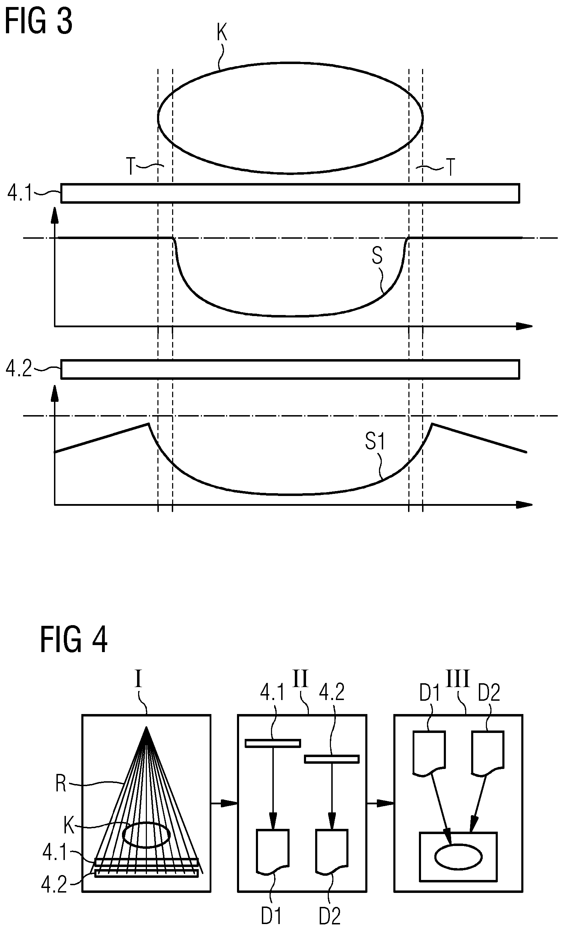

Figur 3 zeigt Datensätze einer Aufnahme gemäß der Erfindung, Es wird hier das gleiche Körperteil aufgenommen, wie in Figur 2 Im Unterschied zu Figur 2 wird nun jedoch mit einer Detektoreinheit 4 aufgenommen, wie sie in Figur 1 zu sehen ist, und welche zwei Detektormodule 4.1, 4.2 umfasst. Der obere Bildteil mit dem ersten Detektormodul 4.1 zeigt als erstes Signal S das gleiche Signal S wie Figur 2, was auch zu erwarten ist, da die Intensität der Strahlung dieselbe ist.

Der untere Bildteil mit dem zweiten Detektormodul 4.2 zeigt jedoch als zweites Signal S1 einen deutlich reduzierten Intensitätsverlauf. Deutlich zu erkennen ist, dass die Intensität im Bereich des Luft-Haut-Übergangs T stark ansteigt, bis über diesen Bereich hinaus und dann abfällt.

Wenn man nun den Ansatz verwendet, dass die beiden Signale S, S1 im Bereich des Körperteils (genauer der beiden inneren gestrichelten Linien) denselben Verlauf zeigen sollten, kann das zweite Signal S1 nun so skaliert werden, dass es zwischen den beiden inneren gestrichelten Linien denselben Verlauf zeigt. Werden die anderen Teile des zweiten Signals entsprechend skaliert, zeigen sie den Verlauf, den das erste Signal eigentlich gehabt haben sollte, wenn das Detektorelement nicht in Sättigung gegangen wäre.

Figur 4 zeigt ein Verfahren zur Aufnahme von Projektionsbildern mit einem Bildgebungssystem wie es z.B. in Figur 1 gezeigt ist.

In Schritt I erfolgt ein Bestrahlen eines Körperteils K eines Patienten P mit der Strahlungsquelle, wobei beide Detektorelemente 4.1, 4.2 der Detektoreinheit 4 durch das Körperteil K hindurch bestrahlt werden.

In Schritt II erfolgt ein Auslesen beider Detektorelemente 4.1, 4.2 und Bilden eines ersten Datensatzes D1 durch Auslese des ersten Detektorelements 4.1 und eines zweiten Datensatzes D2 durch Auslese des zweiten Detektorelements 4.2.

In Schritt III erfolgt ein Erstellung eines Projektionsbildes aus beiden Datensätzen D1, D2, wobei ein zentraler Bereich, dort wo das Körperteil abgebildet wurde, aus dem ersten Datensatz D1 gebildet wird und ein Randbereich, in dem das erste Detektorelement in Sättigung war, aus dem zweiten Datensatz D2 gebildet wird.

Es wird abschließend noch einmal darauf hingewiesen, dass es sich bei der vorhergehend detailliert beschriebenen Erfindung lediglich um Ausführungsbeispiele handelt, welche vom Fach-mann in verschiedenster Weise modifiziert werden können, ohne den Bereich der Erfindung zu verlassen. Weiterhin schließt die Verwendung der unbestimmten Artikel "ein" bzw. "eine" nicht aus, dass die betreffenden Merkmale auch mehrfach vor-handen sein können. Ebenso schließen Begriffe wie "Einheit" nicht aus, dass die betreffenden Komponenten aus mehreren zusammenwirkenden Teil-Komponenten bestehen, die ggf. auch räumlich verteilt sein können. Der Begriff "eine Anzahl" ist als "mindestens ein(e)" zu lesen. Unabhängig vom grammatika-lischen Geschlecht eines bestimmten Begriffes sind Personen mit männlicher, weiblicher oder anderer Geschlechteridentität mit umfasst.

## Patentansprüche

1. Bildgebungssystem (1) zur Aufnahme von Projektionsbildern, das Bildgebungssystem (1) umfassend eine Detektoreinheit (4) und eine Strahlungsquelle (3), wobei die Detektoreinheit (4) zwei hintereinander angeordnete Detektorelemente (4.1, 4.2) aufweist, die beide denselben Aufnahmebereich (A) überdecken und die Strahlungsquelle (3) so eingestellt ist und/oder die Detektoreinheit (4) so ausgestaltet ist, dass für Teile des Aufnahmebereichs (A), in denen bei einer Bestrahlung das erste Detektorelement (4.1) in Sättigung ist, das zweite Detektorelement (4.2) nicht in Sättigung ist, wobei das Bildgebungssystem (1) zusätzlich eine Datenakquisitionseinheit (6) umfasst, die dazu ausgelegt ist, einen ersten Datensatz (D1) des ersten Detektorelements (4.1) aufzunehmen und einen zweiten Datensatz (D2) des zweiten Detektorelements (4.2) aufzunehmen, so dass beide Datensätze (D1, D2) voneinander trennbar sind, **dadurch gekennzeichnet, dass**
das Bildgebungssystem (1) zusätzlich eine Bilderstellungseinheit (8) umfasst, ausgelegt zur Erstellung eines Projektionsbildes, dessen erster Bereich, insbesondere ein zentraler Bereich, durch Daten des ersten Datensatzes (D1) erstellt wird, und dessen zweiter Bereich, insbesondere ein Randbereich, durch Daten des zweiten Datensatzes (D2) oder Daten beider Datensätze (D1, D2) erstellt wird, wobei Bilddaten, bei denen das erste Detektorelement (4.1) in Sättigung war, mittels des zweiten Datensatzes (D2) rekonstruiert werden,
wobei das Bildgebungssystem (1) dazu ausgelegt ist, Position und Signalverlauf an einer Oberfläche eines aufgenommenen Objekts aus dem zweiten Datensatz (D2) zu extrahieren und diese Informationen für eine Bestimmung eines entsprechenden Signalverlaufs aus dem ersten Datensatz (D1) zu verwenden.

2. Bildgebungssystem nach Anspruch 1, wobei die Strahlungsquelle (3) eine Röntgenstrahlenquelle oder Teilchenstrahlenquelle ist, wobei das Bildgebungssystem (1) bevorzugt ein medizintechnisches Bildgebungssystem (1) ist, insbesondere für die Mammographie.

3. Bildgebungssystem nach einem der vorangehenden Ansprüche, umfassend eine Korrektureinheit (7) ausgelegt zur Korrektur zumindest eines der Datensätze (D1, D2), bevorzugt wobei die Korrektureinheit (7) zu mindestens einer der folgenden Korrekturen ausgelegt ist:
- Rauschunterdrückung,
- Pixelregistrierung,
- Korrektur der Kegelstrahlvergrößerung und/oder
- spektrale Korrektur.

4. Bildgebungssystem nach einem der vorangehenden Ansprüche, wobei der erste Bereich durch Daten des ersten Datensatzes (D1) und auch durch Daten des zweiten Datensatzes (D2) erstellt wird.

5. Bildgebungssystem nach einem der vorangehenden Ansprüche, wobei das zweite Detektorelement (4.2) dazu ausgelegt ist, höherenergetische Strahlung zu empfangen als das erste Detektorelement (4.1).

6. Bildgebungssystem nach einem der vorangehenden Ansprüche, wobei das erste Detektorelement (4.1) als Absorber ausgestaltet ist und insbesondere wobei dessen Detektionsschicht oder eine Fläche in oder an einer Detektionsschicht so ausgestaltet ist, dass bei einer vorbestimmten Strahlintensität für eine Bildaufnahme das zweite Detektorelement (4.2) nicht in Sättigung geht,
bevorzugt wobei die Detektoreinheit (4) eine Absorberplatte zwischen dem ersten Detektorelement (4.1) und dem zweiten Detektorelement (4.2) umfasst, welche das zweite Detektorelement (4.2), zumindest teilweise, überdeckt, wobei die Absorberplatte bevorzugt entnehmbar oder austauschbar ist.

7. Verfahren zur Aufnahme von Projektionsbildern mit einem Bildgebungssystem (1) nach einem der vorangehenden Ansprüche, umfassend die Schritte:
- Bestrahlen eines Körperteils (K) eines Patienten mit der Strahlungsquelle (3), wobei beide Detektorelemente (4.1, 4.2) der Detektoreinheit (4) durch das Körperteil (K) hindurch bestrahlt werden,
- Auslesen beider Detektorelemente (4.1, 4.2) und Bilden eines ersten Datensatzes (D1) durch Auslese des ersten Detektorelements (4.1) und eines zweiten Datensatzes (D2) durch Auslese des zweiten Detektorelements (4.2),
- Erstellung eines Projektionsbildes aus beiden Datensätzen (D1, D2), wobei Bildwerte im Projektionsbild, in denen das erste Detektorelement (4.1) beim Auslesen in Sättigung war, auf Daten aus dem zweiten Datensatz (D2) basieren,
**dadurch gekennzeichnet dass**,
Position und Signalverlauf an einer Oberfläche eines aufgenommenen Objekts aus dem zweiten Datensatz (D2) extrahiert werden und diese Informationen für eine Bestimmung eines entsprechenden Signalverlaufs aus dem ersten Datensatz (D1) verwendet werden.

8. Verfahren nach Anspruch 7, wobei ein erster, insbesondere zentraler, Bereich des Projektionsbildes, der das Körperteil (K) zeigt, aus dem ersten Datensatz (D1) und insbesondere auch aus dem zweiten Datensatz (D2), erstellt wird und ein zweiter Bereich, insbesondere ein Randbereich um einen zentralen Bereich herum, aus dem zweiten Datensatz (D2) oder einer Kombination beider Datensätze (D1, D2) erstellt wird, wobei der zweite Datensatz (D2) bevorzugt an den ersten Datensatz (D1) angepasst wird,
bevorzugt wobei der zweite Bereich, insbesondere ein Randbereich, durch eine gewichtete Faltung beider Datensätze (D1, D2) gebildet wird oder der zweite Datensatz (D2) skaliert wird, insbesondere durch Verwendung eines Faltungskerns oder durch Spektralfilterung mit einer anschließenden Wichtung von Daten des zweiten Datensatzes (D2) mit den entsprechenden Daten des ersten Datensatzes (D1),
wobei besonders bevorzugt aus dem zweiten Datensatz (D2) und dem ersten Datensatz (D1) eine Gesamtdosis über das gesamte Bild ermittelt wird.

9. Verfahren nach Anspruch 7 oder 8, wobei Position und Signalverlauf eines Haut-Luft-Übergangs (T), aus dem zweiten Datensatz (D2) extrahiert werden und diese Informationen für eine Bestimmung eines entsprechenden Signalverlaufs aus dem ersten Datensatz (D1) verwendet werden,
wobei bevorzugt eine seitliche Grenze eines aufgenommenen Objekts, insbesondere ein Haut-Luft Übergang (T), basierend auf dem zweiten Datensatz (D2) bestimmt und/oder korrigiert wird.

10. Verfahren nach einem der Ansprüche 7 bis 9, wobei zumindest einer der Datensätze (D1, D2) im Hinblick auf geometrische Effekte im Zusammenhang mit einer Dual-Layer-Technologie korrigiert wird, wobei bevorzugt eine Pixelregistrierung des ersten Datensatzes (D1) auf den zweiten Datensatz (D2) oder umgekehrt durchgeführt wird und/oder eine Korrektur der Kegelstrahlvergrößerung.

11. Verfahren nach einem der Ansprüche 7 bis 10, wobei die aufgenommenen Datensätze (D1, D2) spektral korrigiert werden, insbesondere basierend auf zuvor erfassten Kalibrierungsdaten, bevorzugt wobei eine Korrektur der Strahlaufhärtung durchgeführt wird.

12. Computerprogrammprodukt, umfassend Befehle, die bei der Ausführung des Programms durch einen Computer diesen veranlassen, die Schritte des Verfahrens nach Anspruch 7 bis 11 auszuführen, wobei Schritte zum Bestrahlen oder Auslesen der Aussendung von entsprechenden Steuerbefehlen entsprechen.

13. Computerlesbares Speichermedium, umfassend Befehle, die bei der Ausführung durch einen Computer diesen veranlassen, die Schritte des Verfahrens nach Anspruch 7 bis 11 auszuführen, wobei Schritte zum Bestrahlen oder Auslesen der Aussendung von entsprechenden Steuerbefehlen entsprechen.

## Claims

1. Imaging system (1) for recording projection images, the imaging system (1) comprising a detector unit (4) and a radiation source (3), wherein the detector unit (4) has two detector elements (4.1, 4.2) arranged one behind the other, which both cover the same recording area (A), and the radiation source (3) is configured and/or the detector unit (4) is designed so that the second detector element (4.2) is not saturated for sections of the recording area (A) in which the first detector element (4.1) is saturated during irradiation, wherein the imaging system (1) also comprises a data acquisition unit (6), which is designed to record a first data set (D1) of the first detector element (4.1) and to record a second data set (D2) of the second data element (4.2), so that both data sets (D1, D2) can be separated from one another, **characterised in that** the imaging system (1) additionally comprises an image generating unit (8) designed to generate a projection image, whose first area, especially a central area, is generated using data from the first data set (D1) and, whose second area, especially a peripheral area, is generated using data from the second data set (D2) or data from both data sets (D1, D2), wherein image data for which the first detector element (4.1) was saturated is preferably reconstructed using the second data set (D2),
wherein the imaging system (1) is designed to extract the position and signal path on a surface of a recorded subject from the second data set (D2) and to use this information to determine a corresponding signal path from the first data set (D1).

2. Imaging system according to claim 1, wherein the radiation source (3) is an X-ray source or particle radiation source, wherein the imaging system (1) is preferably a medical technology imaging system (1), especially for mammography.

3. Imaging system according to one of the preceding claims, comprising a correction unit (7) designed to correct at least one of the data sets (D1, D2), wherein the correction unit (7) is preferably designed for at least one of the following corrections:
- noise suppression,
- pixel registration,
- correction of cone beam enlargement and/or
- spectral correction

4. Imaging system according to one of the preceding claims, wherein the first area is generated using data from the first data set (D1) and also using data from the second data set (D2).

5. Imaging system according to one of the preceding claims, wherein the second detector element (4.2) is designed to receive higher energy radiation than the first detector element (4.1).

6. Imaging system according to one of the preceding claims, wherein the first detector element (4.1) is designed as an absorber and in particular wherein its detection layer or a surface in or on a detection layer is designed so that the second detector element (4.2) does not become saturated with a predetermined radiation intensity for image recording, wherein the detector unit (4) preferably includes an absorber plate between the first detector element (4.1) and the second detector element (4.2), which covers, at least partially, the second detector element (4.2), wherein the absorber plate can preferably be removed or replaced.

7. Method for recording projection images with an imaging system (1) according to one of the preceding claims, comprising the steps:
- irradiating a body part (K) of a patient with the radiation source (3), wherein both detector elements (4.1, 4.2) of the detector unit (4) are irradiated through the body part (K),
- reading both detector elements (4.1, 4.2) and forming a first data set (D1) by reading the first detector element (4.1) and a second data set (D2) by reading the second data element (4.2),
- generating a projection image from both data sets (D1, D2), wherein image values in the projection image in which the first detector element (4.1) was saturated during reading are based on data from the second data set (D2),
**characterised in that**
the position and signal path on a surface of a recorded subject are extracted from the second data set (D2), and this information is used to determine a corresponding signal path from the first data set (D1).

8. Method according to claim 7, wherein a first, especially central, area of the projection image that shows the body part (K) is generated from the first data set (D1) and especially also from the second data set (D2), and a second area, especially a peripheral area around a central area, is generated from the second data set (D2) or a combination of both data sets (D1, D2),
wherein the second data set (D2) is preferably adapted to the first data set (D1),
wherein preferably the second area, especially a peripheral area, is formed by means of a weighted convolution of both data sets (D1, D2), or the second data set (D2) is scaled, especially by using a convolution kernel or spectral filtering with a subsequent weighting of data from the second data set (D2) with the corresponding data from the first data set (D1), wherein it is particularly preferable for a total dose over the entire image to be determined from the second data set (D2) and the first data set (D1).

9. Method according to claim 7 or 8, wherein the position and signal path of a skin-air interface (T) are extracted from the second data set (D2) and this information is used to determine a corresponding signal path from the first data set (D1), wherein a lateral limit of a recorded subject, especially a skin-air interface (T), is preferably determined and/or corrected based on the second data set (D2).

10. Method according to one of claims 7 to 9, wherein at least one of the data sets (D1, D2) is corrected with regard to geometric effects related to dual-layer technology, wherein a pixel registration of the first data set (D1) is preferably performed on the second data set (D2) or vice versa and/or a correction of the cone beam enlargement.

11. Method according to one of claims 7 to 10, wherein the recorded data sets (D1, D2) are spectrally corrected, especially based on previously captured calibration data, wherein a correction of beam hardening is preferably performed.

12. Computer program product, comprising commands that, when the program is executed by a computer, trigger the steps of the method as claimed in claim 7 to 11, wherein steps for irradiation or reading correspond to the sending of appropriate control commands.

13. Computer-readable storage medium, comprising commands that, when the program is executed by a computer, trigger it to execute the steps of the method according to claim 7 to 11, wherein steps for irradiation or reading correspond to the sending of appropriate control commands.

## Revendications

1. Système (1) d'imagerie pour l'enregistrement d'images de projection, le système (1) d'imagerie comprenant une unité (4) de détecteur et une source (3) de rayonnement, dans lequel l'unité (4) de détecteur a deux éléments (4.1, 4.2) de détecteur disposés l'un derrière l'autre, les deux recouvrant la même zone (A) d'enregistrement et la source (3) de rayonnement est réglée et/ou l'unité (4) de détecteur est conformée de manière à ce que, pour des parties de la zone (A) d'enregistrement, dans lesquels, lors d'une exposition, le premier élément (4.1) de détecteur est en saturation, le deuxième élément (4.2) de détecteur n'est pas en saturation, dans lequel le système (1) d'imagerie comprend en outre une unité (6) d'acquisition de données, qui est conçue pour saisir un premier ensemble (D1) de données du premier élément (4.1) de détecteur et un deuxième ensemble (D2) de données du deuxième élément (4.2) de détecteur, de manière à ce que les deux ensembles (D1, D2) de données puissent être séparés l'un de l'autre, **caractérisé en ce que**
le système (1) d'imagerie comprend en outre une unité (8) de production d'image conçue pour la production d'une image de projection, dont une première zone, en particulier une zone centrale, est produite par les données du premier ensemble (D1) de données, et dont la deuxième zone, en particulier une zone de bord, est produite par des données du deuxième ensemble (D2) de données ou des données des deux ensembles (D1, D2) de données, dans lequel on reconstruit, au moyen du deuxième ensemble (D2) de données, des données d'image, pour lesquelles le premier élément (4.1) de détecteur était en saturation,
dans lequel le système (1) d'imagerie est conçu pour extraire du deuxième ensemble (D2) de données position et courbe de signal sur une surface d'un objet enregistré et pour utiliser ces informations pour une détermination d'une courbe de signal appropriée, à partir du premier ensemble (D1) de données.

2. Système d'imagerie suivant la revendication 1, dans lequel la source (3) de rayonnement est une source de rayons X ou une source de rayonnement de particules, dans lequel le système (1) d'imagerie est de préférence un système (1) d'imagerie de la technique médicale, notamment pour la mammographie.

3. Système d'imagerie suivant l'une des revendications précédentes,
comprenant une unité (7) de correction conçue pour la correction d'au moins l'un des ensembles (D1, D2) de données, dans lequel, de préférence l'unité (7) de correction est conçue pour au moins l'une des corrections suivantes :
- suppression du bruit,
- enregistrement de pixel,
- correction de l'agrandissement de rayonnement conique, et/ou
- correction spectrale.

4. Système d'imagerie suivant l'une des revendications précédentes,
dans lequel on produit la première zone par des données du premier ensemble (D1) de données et également par des données du deuxième ensemble (D2) de données.

5. Système d'imagerie suivant l'une des revendications précédentes,
dans lequel le deuxième élément (4.2) de détecteur est conçu pour recevoir du rayonnement de plus grande énergie que le premier élément (4.1) de détecteur.

6. Système d'imagerie suivant l'une des revendications précédentes,
dans lequel le premier élément (4.1) de détecteur est conformé en absorbeur et dans lequel, en particulier sa couche de détection ou une surface dans ou sur une couche de détection est conçue de manière à ce que, pour une intensité déterminée à l'avance du rayonnement pour un enregistrement d'image, le deuxième élément (4.2) de détecteur ne passe pas en saturation,
dans lequel, de préférence l'unité (4) de détecteur comprend, entre le premier élément (4.1) de détecteur et le deuxième élément (4.2) de détecteur, une plaque d'absorbeur, qui recouvre au moins en partie le deuxième élément (4.2) de détecteur, dans lequel la plaque d'absorbeur peut être retirée ou remplacée.

7. Procédé d'enregistrement d'images de projection par un système (1) d'imagerie suivant l'une des revendications précédentes, comprenant les stades :
- exposition d'une partie (K) du corps d'un patient à la source (3) de rayonnement, dans lequel on expose deux éléments (4.1, 4.2) de détecteur de l'unité (4) de détecteur à travers la partie (K) du corps,
- lecture des deux éléments (4.1, 4.2) de détecteur et formation d'un premier ensemble (D1) de données par lecture du premier élément (4.1) de détecteur et d'un deuxième ensemble (D2) de données par lecture du deuxième élément (4.2) de détecteur,
- production d'une image de projection à partir des deux ensembles (D1, D2) de données, dans lequel des valeurs d'image dans l'image de projection, dans lesquelles le premier élément (4.1) de détecteur était à saturation à la lecture, reposent sur des données du deuxième ensemble (D2) de données, **caractérisé en ce que**
l'on extrait, du deuxième ensemble (D2) de données, position et courbe de signal sur une surface d'un objet enregistré et on utilise ces informations pour une détermination d'une courbe de signal appropriée à partir du premier ensemble (D1) de données.

8. Procédé suivant la revendication 7, dans lequel on produit une première zone, en particulier centrale, d'image de projection, qui montre la partie (K) du corps, à partir du premier ensemble (D1) de données et en particulier également à partir du deuxième ensemble (D2) de données, et on produit une deuxième zone, en particulier une zone de bord, autour d'une zone centrale, à partir du deuxième ensemble (D2) de données ou d'une combinaison des deux ensembles (D1, D2) de données, dans lequel on adapte le deuxième ensemble (D2) de données, de préférence au premier ensemble (D1) de données,
dans lequel, de préférence on forme la deuxième zone, en particulier une zone de bord, par une convolution pondérée des deux ensembles (D1, D2) de données ou on met à l'échelle le deuxième ensemble (D2) de données, en particulier en utilisant un noyau de convolution ou par filtrage spectral suivi d'une pondération de données du deuxième ensemble (D2) de données par les données appropriées du premier ensemble (D1) de données, dans lequel, d'une manière particulièrement préférée, on détermine une dose d'ensemble sur toute l'image à partir du deuxième ensemble (D2) de données et du premier ensemble (D1) de données.

9. Procédé suivant la revendication 7 ou 8, dans lequel on extrait position et courbe de signal d'une transition (T) peau-air du deuxième ensemble (D2) de données et on utilise cette information pour une détermination d'une courbe de signal appropriée à partir du premier ensemble (D1) de données,
dans lequel, de préférence on détermine et/ou on corrige, sur la base du deuxième ensemble (D2) de données, une limite latérale de l'objet enregistré, en particulier une transition (T) peau-air.

10. Procédé suivant l'une des revendications 7 à 9, dans lequel on corrige, en liaison avec une technologie à couche duale au moins l'un des ensembles (D1, D2) de données en ce qui concerne des effets géométriques, dans lequel, de préférence on effectue un alignement de pixels du premier ensemble (D1) de données sur le deuxième ensemble (D2) de données ou inversement et/ou une correction de l'agrandissement de rayonnement conique.

11. Procédé suivant l'une des revendications 7 à 10, dans lequel on corrige spectralement les ensembles (D1, D2) de données enregistrées, en particulier sur la base de données d'étalonnage saisies précédemment, dans lequel, de préférence, on effectue une correction de la dureté du rayonnement.

12. Produit de programme d'ordinateur comprenant des instructions qui, lors de l'exécution du programme par l'ordinateur, font que celui-ci exécute les stades du procédé suivant la revendication 7 à 11, dans lequel des stades d'exposition ou de lecture correspondent à l'envoi d'instructions de commande appropriées.

13. Support de mémoire exécutable pouvant être exécuté par ordinateur, comprenant des instructions qui, lors de l'exécution par ordinateur, font que celles-ci exécutent les stades du procédé de la revendication 7 à 11, dans lequel des stades d'exposition ou de lecture correspondent à l'envoi d'instructions de commande appropriées.
